# EUROPEAN PATENT SPECIFICATION

(11) **EP 3 826 581 B1**
(45) Date of publication and mention of the grant of the patent: **31.08.2022**
(21) Application number: 19765559.0
(22) Date of filing: 25.07.2019
(51) Int. Cl.: A61F 2/00

(54) **PARIETAL SUPPORT PROSTHESIS, IN PARTICULAR FOR PELVIC PROLAPSES**
PARIETALE STÜTZPROTHESE, INSBESONDERE FÜR BECKENBODENPROLAPS
PROTHÈSE DE SOUTIEN PARIÉTAL, EN PARTICULIER POUR DES PROLAPSUS PELVIENS

(30) Priority: 25.07.2018 IT 201800007499
(43) Date of publication of application: 02.06.2021
(73) Proprietor: Angiologica B.M. S.R.L., 27028 San Martino Siccomario (PV) (IT)
(72) Inventor: ZONTA, Sandro, 27028 San Martino Siccomario (PV) (IT)
(74) Representative: Cernuzzi, Daniele
(86) International application number: PCT/IB2019/056353
(87) International publication number: WO 2020/021484

(56) References cited:
- WO-A1-2011/003422
- WO-A2-2006/108964
- US-A1- 2003 065 402
- US-A1- 2009 149 700
- US-A1- 2014 121 454
- US-A1- 2015 216 646
- US-A1- 2016 030 148

## Description

### CROSS-REFERENCE TO RELATED APPLICATIONS

This patent application claims priority from Italian patent application no. 102018000007499 filed on 25/07/2018.

### TECHNICAL FIELD

The present invention relates to a support parietal prosthesis. The support parietal prosthesis in accordance with the invention is preferably applied in the treatment of pelvic prolapses, being intended to support internal organs of the lower part of the abdomen (rectum, uterus/vagina, bladder). However, the prosthesis of the invention can also be used in other applications.

### BACKGROUND ART

The pelvic floor is the structure that holds the uterus, vagina, bladder and rectum in place inside the pelvis.

Collapse of the pelvic floor can lead to prolapse of the organs it supports. Lack of support of the pelvic organs means that these no longer remain in their normal position, causing repercussions on their functioning.

To solve this, the use of support parietal prostheses which are inserted into the abdomen is known.

Typically, parietal prostheses consist of meshes made of polymer material, suitably shaped according to their specific use. Parietal prostheses are positioned, also using laparoscopy, on the damaged tissue and, if necessary, secured (sutured) to the surrounding tissues.

Although known parietal prostheses are in general functional and efficient, they can give rise to some problems.

In fact, known parietal prostheses generally have a non-negligible two-dimensional extension, so that they must be folded or wound to be inserted using special surgical instruments, but also tend to assume unwanted positions and configurations before being correctly positioned and secured. Therefore, operations for the insertion and correct positioning of prostheses can pose some difficulties, due to the dimensions and shapes of the prostheses, and to the flexibility of the mesh with which they are made; in particular, the presence of tabs and wings of various extension can make handling of prostheses difficult, reducing visibility on the surrounding tissues and prolonging operating times.

Moreover, the materials with which parietal prostheses are made may not be completely satisfactory. For example, it has been observed that certain materials widely used in the field, such as polypropylene, can give rise to undesirable reactions by the body, with phenomena of inflammation and adhesions. On the other hand, the use of different materials can compromise the mechanical properties of prostheses.

Document US2009149700 discloses an adjustable-length support sling assembly for internal placement within a patient including a generally flat sling member formed of a mesh material. Each respective end of the sling member is permanently affixed to a corresponding anchor member. A portion of the sling member adjacent each respective end is folded upon itself by a predetermined length and releasably secured within an annular slot in the corresponding anchor member.

The dimensions of each respective annular slot is selected to retain the corresponding folded portion of the sling member within the slot until sufficient tension is applied by the sling member that exceeds a respective predetermined tension level.

WO2006/108964 describes an arrangement for surgically treating a prolapse affecting a protruding organ consisting of an implant comprising a base for supporting the organ and four suspension arms. The implant comprises a pocket which consists of an insulating wall and is used for receiving the base for supporting the organ and the suspension arms thereof in a folded position on the support base in such a way that at least one traction element penetrates the wall and remains outside of the pocket.

US2015216646 and US2003065402 describe other examples of slings. US2016030148 discloses an implantable reinforcement prosthesis, in particular for reinforcing the abdominal wall.

### DISCLOSURE OF INVENTION

An object of the present invention is to provide a support parietal prosthesis that is free from the drawbacks of the prior art mentioned above; in particular, an object of the invention is to provide a support parietal prosthesis that can be simply and efficiently implanted in the correct position and conformation.

It is a further object of the invention to provide a support parietal prosthesis that has suitable mechanical characteristics, is relatively simple and inexpensive to produce and at the same time is able to reduce adverse reactions by the body.

The present invention therefore relates to a support parietal prosthesis, the essential terms of which are defined in the appended claim 1, and the additional features are defined in the dependent claims.

In substance, the support parietal prosthesis of the invention is arranged, before implantation, in an insertion operating position in which the prosthesis has greatly reduced spatial dimensions with respect to the use operating position, which it assumes after implantation. In this way, it is easier to insert the prosthesis in the operation area, for example using the laparoscopic technique. Once inserted, the prosthesis can then be taken to its use operating position in a very simple and rapid manner.

In particular, the prosthesis in the insertion operating position does not have flexible tabs of large dimensions that can be difficult to orientate correctly and that in any case can compromise the visibility of the surrounding tissues during surgery.

Therefore, insertion of the prosthesis, in particular using the laparoscopic technique, is greatly simplified, improving the operator's visibility of the surrounding tissues and hence also reducing the operating times.

According to a further aspect of the invention, the prosthesis is made of a mesh of a material such as polypropylene, polyester or PTFE, having suitable mechanical characteristics, in particular in terms of rigidity and elasticity, but also has, in the area destined to come into contact with the tissue wall to be supported, a contact layer made of a different material, in particular a material such as polyurethane, having low adhesion and low inflammatory activity on the tissues with which it comes into contact.

### BRIEF DESCRIPTION OF THE DRAWINGS

Further characteristics and advantages of the present invention will be apparent from the description below of a non-limiting example of embodiment thereof, with reference to the figures of the accompanying drawings, in which:
- Fig. 1 is a top plan view of a support parietal prosthesis in accordance with the invention, shown in a use operating position;
- Fig. 2 is a top plan view of the prosthesis of Fig. 1, shown in an insertion operating position;
- Fig. 3 is a perspective view of the prosthesis of Fig. 1, shown in an intermediate operating position;
- Fig. 4 is a schematic sectional view along the plane IV-IV of Fig. 2 and with parts not to scale.

### BEST MODE FOR CARRYING OUT THE INVENTION

In the accompanying figures, a support parietal prosthesis, in particular for pelvic prolapses, is indicated as a whole with 1. It is understood that the prosthesis 1 can have other uses.

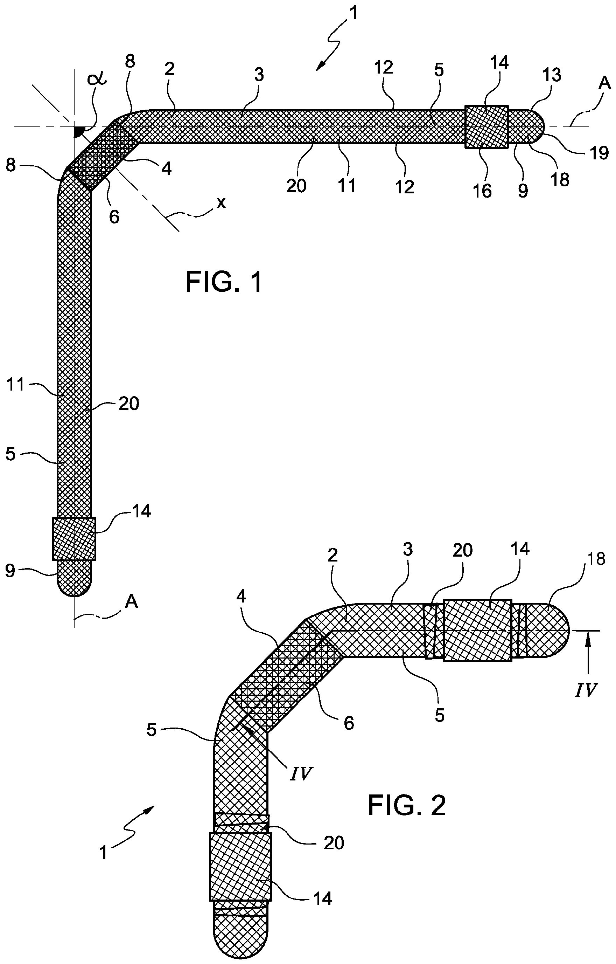

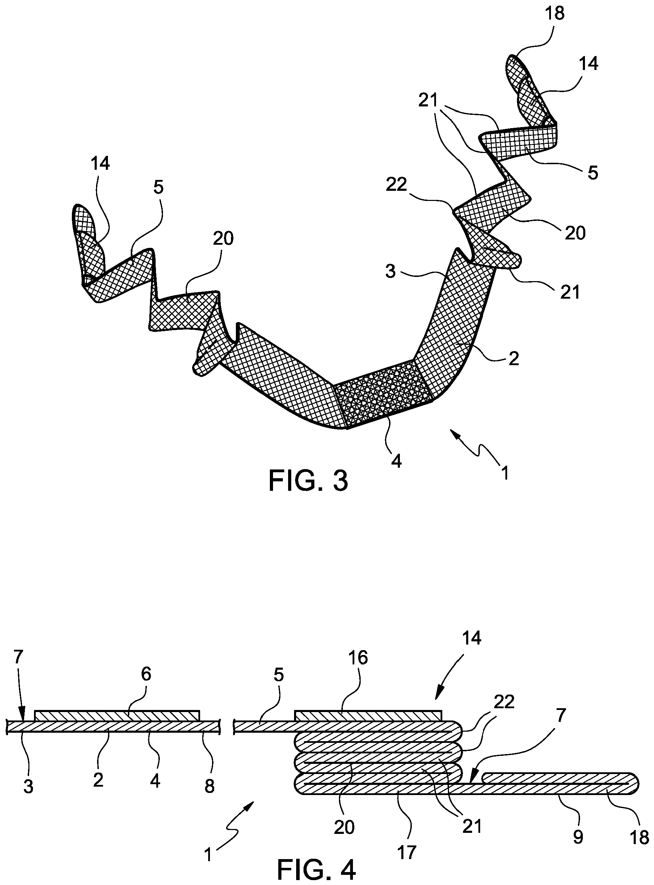

In Figs. 1-3, the prosthesis 1 is illustrated in respective operating positions and precisely in a use operating position (Fig. 1), in an insertion operating position (Fig. 2), and in an intermediate operating position (Fig. 3).

In particular, Fig. 1 shows the prosthesis 1 in a use operating position, which the prosthesis 1 assumes when it is produced and, ideally, in use, i.e., when it is implanted in a patient (clearly, the prosthesis 1 is shown in Fig. 1 in a form lying completely on a plane, while in actual use the prosthesis 1 can have a three-dimensional extension that follows the tissues and the organs on which it rests). In Fig. 2, the prosthesis 1 is illustrated in an insertion operating position, which is the position of the prosthesis 1 ready to be inserted into the patient, for example using a laparoscopic instrument (trocar). In Fig. 3, the prosthesis 1 is shown in an (illustrative) intermediate operating position, which the prosthesis 1 assumes passing from the insertion operating position to the use operating position.

The prosthesis 1 has a flat sheet body 2, defined by a mesh 3 made of suitably shaped polymer material.

In the embodiment described and illustrated herein, provided purely by way of example, the sheet body 2 is a single-layer body, formed by a single layer of mesh material. It is understood that the sheet body 2, or parts thereof, can have a multi-layer structure.

The mesh 3 can have a different structure (woven or interlaced) and can be made of various materials, for example polypropylene, polyester, PTFE, etc.

The sheet body 2 comprises a central support portion 4, for example (but not necessarily) substantially quadrangular, destined in use to support a damaged or weakened parietal tissue, and a pair of wings 5 extending from the central portion 4 substantially along respective longitudinal axes A.

The central portion 4 is provided with a contact layer 6, positioned on a face 7 of the sheet body 2 and made of a polymer material different from the material of the mesh 3. In particular, the contact layer 6 is made of a material, such as polyurethane, having low adhesion and low inflammatory activity on the tissues with which it comes into contact. The contact layer 6 is secured to the underlying central portion 4 of the mesh 3 in a known way, for example by heat-sealing (possibly only along respective lateral edges).

In the embodiment shown, the wings 5 extend from respective opposite sides of the central portion 4 and diverge from the central portion 4 and are symmetrical with respect to a middle axis X of the central portion 4.

In particular, the wings 5 extend along respective axes A inclined with respect to one another and with respect to the axis X. For example, the axes A of the wings 5 form an angle α comprised between around 60° and around 120°. In the embodiment shown, for example, the angle α between the axes A is of around 90°.

The wings 5 extend longitudinally between respective root ends 8, joined to the central portion 4, and respective free ends 9, opposite to the root ends 8.

The wings 5 diverge from the central portion 4 towards the respective free ends 9.

Each wing 5 is defined by a band 11 longitudinally elongated along the axis A and, for example, having a substantially rectangular shape.

It is understood that the shape of the central portion 4 and of the wings 5, and also the inclination of the wings 5 with respect to the central portion 4, and the overall shape of the prosthesis 1, can differ from what is here described and illustrated purely by way of example.

Each band 11 has two opposite longitudinal edges 12, for example (but not necessarily) substantially rectilinear and parallel, and a front edge 13, positioned at the respective free end 9 and preferably rounded and/or bevelled.

Each wing 5 is provided with a pocket 14, for example (but not necessarily) positioned in proximity of the free end 9 and spaced apart from the free end 9.

The pockets 14 are in particular tubular pockets extending along the respective axes A.

Each pocket 14 is closed around the respective axis A and is axially open along the axis A, having open opposite axial ends.

Preferably, each pocket 14 is formed by a lateral flap 16 of the mesh 3 projecting laterally from a longitudinal edge 12 of a band 11 and is folded to 180° on the band 11 and is joined, for example heat-sealed, to the opposite longitudinal edge 12. Therefore, the pocket 14 has a pair of opposite walls facing and joined to one another along the longitudinal edges 12 and defined by a portion 17 of the band 11 and by the flap 16 (Fig. 4).

The flaps 16 are preferably folded on a same face of the sheet body 2, in particular the face 7 on which the contact layer 6 is also optionally positioned. Consequently, both pockets 14 are arranged on a same face, in particular the face 7, of the sheet body 2.

Each wing 5 is provided with a grip portion 18 positioned at the free end 5 and protruding from the pocket 14; the grip portion 18 has a peripheral edge 19 including the front edge 13 and preferably rounded or bevelled.

Preferably, the grip portion 18 is a reinforced portion, for example consisting of two (or more) layers of mesh 3 superimposed and joined to one another, for example welded along the peripheral edge 19.

Each wing 5 comprises an operative stretch 20 that, in the use operating position of the prosthesis 1 (Fig. 1) extends longitudinally along the axis A from the respective pocket 14 towards the central portion 4 for a predetermined (axial) length.

In accordance with the invention, the stretch 20 selectively assumes: an extended configuration (shown in Fig. 1), in which the stretch 20 has a substantially planar and substantially linear shape along the axis A, in particular elongated longitudinally along the axis A and for example substantially rectilinear; and a retracted configuration (shown in Figs. 2 and 4), in which the stretch 20 is accordion-folded along the axis A and has a plurality of superimposed portions 21 alternately folded in opposite directions, joined to one another by folds 22.

As shown in Fig. 1, the stretches 20 are in the respective extended configurations when the prosthesis 1 assumes the use operating position, i.e., when the prosthesis 1 is implanted in a patient: the stretches 20 are lying substantially on a plane, defined by the sheet body 2, and along the respective axes A.

Instead, the stretches 20 are in the respective retracted configurations when the prosthesis 1 assumes the insertion operating position (Fig. 2), having reduced dimensions and used to facilitate insertion of the prosthesis 1, for example using a laparoscopic instrument (trocar).

Each stretch 20 in its retracted configuration has, starting from the pocket 14, a first portion 21 joined to the portion 17 and folded towards the pocket 14, and further successive portions 21 alternately zig zag folded and joined to one another by respective folds 22 transverse (in particular orthogonal) to the axis A. The stretch 20 comprises a plurality of opposite folds 22.

The portions 21 are arranged one on top of the other and are all positioned above the face 7.

Each stretch 20, in its retracted configuration, is housed in the respective pocket 14. In particular, the portions 21 are inserted, at least partially, in the pocket 14.

When the prosthesis 1 is manufactured, for example starting from a sheet of mesh material that is cut to form the sheet body 2, the stretches 20 are in their extended configurations and the prosthesis 1 as a whole is in the use operating position (Fig. 1) .

Manufacture of the prosthesis 1 is completed by forming the pockets 14, the grip portions 18 and applying the contact layer 6.

As already mentioned, the pockets 14 are advantageously made by folding the flaps 16 on the respective wings 5 and securing their ends to the respective longitudinal edges 12 (for example by heat-sealing).

The grip portions 18 are, for example, made by folding and welding two layers of mesh 3 one on top of the other, and optionally trimming the peripheral edges 19.

The contact layer 6 is applied to the central portion 4, for example, by heat-sealing along respective lateral edges.

To facilitate insertion and handling of the prosthesis 1, in particular using a trocar or other laparoscopic instrument, the prosthesis 1 is arranged with the stretches 20 of the wings 5 in the respective retracted configurations.

As schematically illustrated in Figs. 2, 3 and 4, the stretches 20 are then folded, superimposing on one another the portions 21 folded along the folds 22, and inserted in the respective pockets 14 (it is understood that it would also be possible to first fold the stretches 20 and then form the pockets around the folded stretches 20).

When the prosthesis 1 has the operative stretches 20 of the wings 5 in retracted configuration, the prosthesis 1 has significantly reduced dimensions and can be more easily inserted into the operation area.

Insertion of the prosthesis 1, in particular using the laparoscopic technique, is thus greatly simplified, improving the operator's visibility of the surrounding tissues and hence also reducing the operation times.

Once the prosthesis 1 has been inserted, the stretches 20 of the wings 5 are extracted from the pockets 14, pulling the grip portions 18, and assume their extended configurations.

At this point, the wings 5 can be secured, for example sutured, to the surrounding tissues. Optionally, the free ends 9 of the wings 5, including the pockets 14, are cut and removed.

Finally, it is understood that further modifications and variants can be made to the support parietal prosthesis without departing from the scope of the appended claims.

## Claims

1. A support parietal prosthesis (1), in particular for pelvic prolapses, having a sheet body (2), defined by a mesh (3) and comprising a central portion (4) and at least a wing (5) extending from the central portion (4) substantially along an axis (A) and provided with a pocket (14) which, in an insertion operating position of the prosthesis (1), houses an operative stretch (20) of the wing (5), accordion-folded in a retracted configuration and extractable from the pocket (14) to assume, in a use operating position of the prosthesis (1), an extended configuration longitudinally elongated along the axis (A), wherein the pocket (14) is a tubular pocket closed around the axis (A) and open along the axis (A), and the wing (5) is provided with a grip portion (18) positioned at a free end (9) of the wing (5) a; the prosthesis being **characterised in that** the pocket (14) has open opposite axial ends, and **in that** the grip portion of the wing is protruding from the pocket (14).

2. The prosthesis according to claim 1, wherein the stretch (20) has, in the extended configuration, a shape substantially planar and substantially linear along the axis (A); and in the retracted configuration, the stretch (20) has a plurality of superimposed portions (21) alternately folded in opposite directions, connected to one another by folds (22) transversal to the axis (A) and inserted, at least partly, in the pocket (14).

3. The prosthesis according to claim 2, wherein the stretch (20) comprises a plurality of opposite folds (22).

4. The prosthesis according to one of the preceding claims, wherein the pocket (14) is positioned in proximity of a free end (9) of the wing (5) and is axially spaced apart from said free end (9).

5. The prosthesis according to one of the preceding claims, wherein the grip portion (18) has a rounded or bevelled peripheral edge (19).

6. The prosthesis according to claim 5, wherein the grip portion (18) is a reinforced portion, consisting of two or more layers of the mesh (3), superimposed and joined to one another.

7. The prosthesis according to one of the preceding claims, wherein the wing (5) is defined by a band (11) longitudinally elongated along the axis (A), having two opposite longitudinal edges (12) and a front edge (13), positioned at the free end (9) of the wing (5); and the pocket (14) is formed by a lateral flap (16) of mesh (3) projecting laterally from a first longitudinal edge (12) of the band (11) and folded on the band (11) and joined to a second longitudinal edge (12) of the band (11).

8. The prosthesis according to one of the preceding claims, comprising a pair of wings (5) extending from the central portion (4) substantially along respective axes (A) and provided with respective pockets (14) housing respective operative stretches (20) of the wings (5), accordion-folded in respective retracted configurations and extractable from the pockets (14) to assume respective extended configurations longitudinally elongated along the respective axes (A).

9. The prosthesis according to claim 8, wherein the pockets (14) are arranged on a same face (7) of the sheet body (2).

10. The prosthesis according to claim 8 or 9, wherein the wings (5) extend from respective opposite sides of the central portion (4) and diverge from the central portion (4) towards respective free ends (9), and are symmetric with respect to a centreline axis (X) of the central portion (4).

11. The prosthesis according to one of the preceding claims, wherein the mesh (3) is made of a first polymer material, in particular polypropylene, polyester or PTFE, and the central portion (4) is provided with a contact layer (6), positioned on a face (7) of the sheet body (2) and made of a second polymer material different from the material of the mesh (3) and having low adhesion and low inflammatory activity on tissues with which it comes into contact, in particular polyurethane.

## Patentansprüche

1. Parietale Stützprothese (1), insbesondere für Beckenbodensenkungen, die einen Folienkörper (2) aufweist, der durch ein Gewebe (3) definiert ist und einen Mittelabschnitt (4) und mindestens einen Flügel (5), der sich vom Mittelabschnitt (4) im Wesentlichen entlang einer Achse (A) erstreckt und mit einer Tasche (14) versehen ist, die in einer Einsetzarbeitsposition der Prothese (1) ein funktionstechnisches Dehnelement (20) des Flügels (5) aufnimmt, das in einer eingezogenen Konfiguration akkordeonartig gefaltet und aus der Tasche (14) extrahierbar ist, derart, dass es in einer Gebrauchsarbeitsposition der Prothese (1) eine gestreckte Konfiguration annimmt, die sich in Längsrichtung entlang der Achse (A) erstreckt, umfasst, wobei die Tasche (14) eine schlauchförmige Tasche ist, die um die Achse (A) geschlossen und entlang der Achse (A) offen ist, und der Flügel (5) mit einem Greifabschnitt (18) versehen ist, der an einem freien Ende (9) des Flügels (5) angeordnet ist; wobei die Prothese **dadurch gekennzeichnet ist, dass** die Tasche (14) offene gegenüberliegende axiale Enden aufweist und dass der Greifabschnitt des Flügels aus der Tasche (14) vorsteht.

2. Prothese nach Anspruch 1, wobei das Dehnelement (20) in der gestreckten Konfiguration eine Form aufweist, die im Wesentlichen eben und im Wesentlichen entlang der Achse (A) geradlinig ist; und das Dehnelement (20) in der eingezogenen Konfiguration mehrere überlagerte Abschnitte (21) aufweist, die abwechselnd in entgegengesetzten Richtungen gefaltet sind, durch Falze (22) quer zur Achse (A) miteinander verbunden sind und zumindest teilweise in die Tasche (14) eingesetzt sind.

3. Prothese nach Anspruch 2, wobei das Dehnelement (20) mehrere entgegengesetzte Falze (22) umfasst.

4. Prothese nach einem der vorhergehenden Ansprüche, wobei die Tasche (14) in räumlicher Nähe eines freien Endes (9) des Flügels (5) angeordnet ist und von dem freien Ende (9) axial beabstandet ist.

5. Prothese nach einem der vorhergehenden Ansprüche, wobei der Greifabschnitt (18) eine abgerundete oder angeschrägte Außenkante (19) aufweist.

6. Prothese nach Anspruch 5, wobei der Greifabschnitt (18) ein verstärkter Abschnitt ist, der aus zwei oder mehr Schichten des Gewebes (3) besteht, die einander überlagert und miteinander verbunden sind.

7. Prothese nach einem der vorhergehenden Ansprüche, wobei der Flügel (5) durch einen Streifen (11) definiert ist, der sich in Längsrichtung entlang der Achse (A) erstreckt, wobei er zwei gegenüberliegende Längskanten (12) und eine Vorderkante (13), die an dem freien Ende (9) des Flügels (5) angeordnet ist, aufweist; und die Tasche (14) durch eine seitliche Lasche (16) aus Gewebe (3) gebildet ist, die von einer ersten Längskante (12) des Streifens (11) seitlich vorsteht und auf dem Streifen (11) gefaltet und mit einer zweiten Längskante (12) des Streifens (11) verbunden ist.

8. Prothese nach einem der vorhergehenden Ansprüche, die ein Paar Flügel (5) umfasst, die sich vom Mittelabschnitt (4) im Wesentlichen entlang jeweiliger Achsen (A) erstrecken und mit jeweiligen Taschen (14) versehen sind, die jeweilige funktionstechnische Dehnelemente (20) der Flügel (5) aufnimmt, die in jeweiligen eingezogenen Konfigurationen akkordeonartig gefaltet und aus den Taschen (14) extrahierbar sind, derart, dass sie jeweilige gestreckte Konfigurationen annehmen, die sich in Längsrichtung entlang der jeweiligen Achsen (A) erstrecken.

9. Prothese nach Anspruch 8, wobei die Taschen (14) auf derselben Fläche (7) des Folienkörpers (2) angeordnet sind.

10. Prothese nach Anspruch 8 oder 9, wobei sich die Flügel (5) von jeweiligen entgegengesetzten Seiten des Mittelabschnitts (4) erstrecken und vom Mittelabschnitt (4) in Richtung jeweiliger freier Enden (9) auseinanderlaufen und in Bezug auf eine Mittellinienachse (X) des Mittelabschnitts (4) symmetrisch sind.

11. Prothese nach einem der vorhergehenden Ansprüche, wobei das Gewebe (3) aus einem ersten Polymermaterial, insbesondere Polypropylen, Polyester oder PTFE, hergestellt ist und der Mittelabschnitt (4) mit einer Kontaktschicht (6) versehen ist, die auf einer Fläche (7) des Folienkörpers (2) angeordnet ist und aus einem zweiten Polymermaterial, das von dem Material des Gewebes (3) verschieden ist und auf biologischem Gewebe, mit dem es in Kontakt gelangt, ein geringes Haftvermögen und eine niedrige entzündliche Aktivität aufweist, insbesondere Polyurethan, hergestellt ist.

## Revendications

1. Prothèse de soutien pariétal (1), en particulier pour prolapsus pelviens, possédant un corps en feuille (2), défini par un maillage (3) et comprenant une partie centrale (4) et au moins une aile (5) s'étendant à partir de la partie centrale (4) sensiblement le long d'un axe (A) et pourvue d'une poche (14) qui, dans une position fonctionnelle d'insertion de la prothèse (1), loge une extension active (20) de l'aile (5), pliée en accordéon dans une configuration rétractée et pouvant être extraite de la poche (14) pour prendre, dans une position fonctionnelle d'utilisation de la prothèse (1), une configuration étendue longitudinalement allongée le long de l'axe (A), dans laquelle la poche (14) est une poche tubulaire fermée autour de l'axe (A) et ouverte le long de l'axe (A), et l'aile (5) est pourvue d'une partie de préhension (18) positionnée au niveau d'une extrémité libre (9) de l'aile (5) ; la prothèse étant **caractérisée en ce que** la poche (14) présente des extrémités axiales opposées ouvertes, et **en ce que** la partie de préhension de l'aile fait saillie depuis la poche (14).

2. Prothèse selon la revendication 1, dans laquelle l'extension (20) présente, dans la configuration étendue, une forme sensiblement plane et sensiblement linéaire le long de l'axe (A) ; et dans la configuration rétractée, l'extension (20) comporte une pluralité de parties superposées (21) pliées en alternance dans des directions opposées, reliées entre elles par des plis (22) transversaux à l'axe (A) et insérées, au moins en partie, dans la poche (14).

3. Prothèse selon la revendication 2, dans laquelle l'extension (20) comprend une pluralité de plis (22) opposés.

4. Prothèse selon l'une des revendications précédentes, dans laquelle la poche (14) est positionnée à proximité d'une extrémité libre (9) de l'aile (5) et est axialement espacée de ladite extrémité libre (9).

5. Prothèse selon l'une des revendications précédentes, dans laquelle la partie de préhension (18) comporte un bord périphérique arrondi ou en biseau (19).

6. Prothèse selon la revendication 5, dans laquelle la partie de préhension (18) est une partie renforcée, constituée de deux couches du maillage (3) ou plus, superposées et jointes entre elles.

7. Prothèse selon l'une des revendications précédentes, dans laquelle l'aile (5) est définie par une bande (11) allongée longitudinalement le long de l'axe (A), comportant deux bords longitudinaux (12) opposés et un bord avant (13), positionné au niveau de l'extrémité libre (9) de l'aile (5) ; et la poche (14) est formée par un rabat latéral (16) du maillage (3) se projetant latéralement depuis un premier bord longitudinal (12) de la bande (11) et plié sur la bande (11) et joint à un second bord longitudinal (12) de la bande (11).

8. Prothèse selon l'une des revendications précédentes, comprenant une paire d'ailes (5) s'étendant depuis la partie centrale (4) sensiblement le long d'axes (A) respectifs et pourvues de poches (14) respectives logeant des extensions actives (20) respectives des ailes (5), pliées en accordéon dans des configurations rétractées respectives et pouvant être extraites des poches (14) pour prendre des configurations étendues longitudinalement allongées respectives le long des axes (A) respectifs.

9. Prothèse selon la revendication 8, dans laquelle les poches (14) sont agencées sur une même face (7) du corps de feuille (2).

10. Prothèse selon la revendication 8 ou 9, dans laquelle les ailes (5) s'étendent depuis des côtés opposés respectifs de la partie centrale (4) et divergent depuis la parie centrale (4) vers des extrémités libres (9) respectives, et sont symétriques par rapport à un axe de ligne médiane (X) de la partie centrale (4).

11. Prothèse selon l'une des revendications précédentes, dans laquelle le maillage (3) est composé d'un premier matériau polymère, en particulier du polypropylène, du polyester ou PTFE, et la partie centrale (4) est pourvue d'une couche de contact (6), positionnée sur une face (7) du corps en feuille (2) et composée d'un second matériau polymère différent du matériau du maillage (3) et présentant une faible adhérence et une faible activité inflammatoire sur des tissus avec lesquels il entre en contact, en particulier du polyuréthane.
